# EUROPEAN PATENT SPECIFICATION

(11) **EP 3 537 949 B1**
(45) Date of publication and mention of the grant of the patent: **16.02.2022**
(21) Application number: 17808668.2
(22) Date of filing: 10.11.2017
(51) Int. Cl.: A61B 1/307, A61B 1/005, A61B 1/00, A61B 1/05, A61B 1/06

(54) **DISPOSABLE MEDICAL SYSTEMS AND DEVICES**
MEDIZINISCHE EINWEGSYSTEME UND VORRICHTUNGEN
SYSTÈMES MÉDICAUX JETABLES ET DISPOSITIFS

(30) Priority: 11.11.2016 US 201662420938 P
(43) Date of publication of application: 18.09.2019
(73) Proprietor: Boston Scientific Scimed, Inc., Maple Grove, MN 55311 (US)
(72) Inventor: REEVER, Kenneth, Hopedale MA 01747 (US); TAH, Richard, Milford MA 01757 (US); MACLEAN, Brian, Westford MA 01886 (US); ZAPPIA, Thomas, West Boylston MA 01583 (US); MAILLET, Maria, Hudson MA 01749 (US); SOKOLOVSKIY, Zakh, Brighton MA 02135 (US); BOROS, Peter, Ellettsville IN 47429 (US); ARRUDA, Joseph, Taunton MA 02780 (US)
(74) Representative: Pfenning, Meinig & Partner mbB
(86) International application number: PCT/US2017/061069
(87) International publication number: WO 2018/089770

(56) References cited:
- WO-A2-2012/151073
- US-A- 6 095 970
- US-A1- 2011 009 694
- US-A1- 2012 209 071
- US-A1- 2013 172 670
- US-A1- 2015 342 446

## Description

### Cross-Reference To Related Applications

This patent application claims the benefit of priority under 35 U.S.C. § 119 to U.S. Provisional Patent Application No. 62/420,938, filed November 11, 2016.

### Technical Field

Various aspects of the present disclosure relate generally to medical systems and devices. More specifically, the present disclosure relates to disposable medical systems and/or devices.

### Background

The field of endoscopy covers systems and methods a user may employ to examine and/or treat a subject with, e.g., the assistance of an endoscope or other suitable introduction sheaths or devices. An endoscope (or other suitable introduction device) may provide for viewing of, for example, the interior of a hollow organ or cavity in the subject's body (e.g., the gastrointestinal (GI) tract, the urinary tract, the respiratory tract, etc.). Ureteroscopy, for example, is a subset of endoscopy. Ureteroscopy may include procedures in which an endoscope (such as, e.g., a ureteroscope, uteroscope, cystoscope, hysteroscope, etc.) may be passed through the subject's urethra and bladder, and directly into the subject's ureter. The endoscope may be further inserted into the subject's kidney for pyeloscopic procedures. These procedures may be useful in the diagnosis and the treatment of disorders of the subject's urinary tract, such as the presence of kidney stones that may block urinary tract ducts.

Sterilization of such endoscopes is imperative to avoid cross-contamination between patients. Unfortunately, insufficient sterilization of endoscopes is a persistent, and hazardous problem in the medical device industry. Not only is it difficult for hospitals and medical professionals to reprocess or sterilize a wide array of endoscopes effectively, it's also costly. Moreover, typical endoscopes are expensive to manufacture, requiring highly precise and often individual endoscope-specific components (e.g., camera, illumination features, various medical tool adaptors, etc.) resulting in devices exceeding tens of thousands, and possibly hundreds of thousands of dollars. Thus, such devices are impractical for use as a one-time-use disposable device.

The systems and devices of the current disclosure may rectify some of the deficiencies described above or address other aspects of the prior art.

Document WO 2012/151073 A2 describes methods and devices for performing a combined hysteroscopy and endometrial sampling. In particular, techniques for improving visual images include forward facing fluid ports for clearing tissue debris and LED positioning and design are described. Tip and shaft pieces are formed separately in order to improve manufacturability. User interface features are described including handle-mounted buttons as well the use of an interactive integrated touch screen display. The handle and display can be mated to a docking station for storage and recharging batteries.

Document US 2015/342446 A2 describes an endoscope assembly which comprises at least one front-pointing camera and at least one front illuminator associated therewith, at least one side-pointing camera and at least one of side illuminator associated therewith, a first front working channel configured for insertion of a medical tool and a second front working channel configured for insertion of a medical tool.

Document US 2013/172670 A1 describes an endoscope which comprises an elongated shaft terminating with a tip section wherein. Said tip section comprises a permanent section connected to the elongated shaft and a removable section securely connectable to the permanent section. The removable section comprises at least one capture device and at least one light source.

Document US 2012/209071 A1 describes an endoscope which includes a detachable wireless imaging device and an insertion tube having a distal end region. The attachment of the detachable wireless imaging device detachably attaches the detachable wireless imaging device to the distal end region of the insertion tube.

### SUMMARY

Examples of the present disclosure relate to medical devices.

In one example, an insertion device includes a hollow handle and a shaft extending distally of the hollow handle. The insertion device also includes a cap coupled to a distal end of the shaft. The cap includes a camera and an LED coupled thereto.

Examples of the insertion device include one or more of the following features. The shaft is a rigid shaft. The shaft includes a multi-lumen extrusion. The shaft includes a plurality of discrete lumens fixedly coupled together. The hollow handle includes a rounded ball-head and an extension depending therefrom. The shaft extends from a location within the ball-head and through a side of the ball-head of the hollow handle. A port extends from a side of the ball-head of the hollow handle opposite the side from which the shaft extends. The hollow handle includes a plug extending from a portion of the extension of the hollow handle opposite the ball-head. The plug is selectively coupleable and uncouplable from an umbilicus connector. The handle includes two fluid ports proximate the plug. Each of the two fluid ports is fluidly coupled to a tube extending through the hollow handle, through the shaft, and toward the cap. The umbilicus is coupled to a computer. The LED is a first LED, the insertion device further includes a second LED disposed on the cap. Each of the first and second LEDs is disposed on opposing sides of the camera. A working channel extends through and proximally of a proximal end of the shaft.

In a further example, an insertion device consists essentially of a handle including a head having a port which extends from a first side of the head and a grip which depends from the head. The insertion device also includes a shaft extending distally of the hollow handle and a cap coupled to a distal end of the shaft. The cap includes a camera and a light sourced coupled thereto.

Examples of the insertion device include one or more of the following features. A proximal end of the shaft is disposed within the rounded ball-head of the handle. A plug extends from the grip in a direction opposite of the ball-head, and the plug is selectively coupleable and uncouplable from an umbilicus connector. The grip includes two ports proximate the plug, and each of the two ports are fluidly coupled to a tube extending through the handle, through the shaft, and toward the cap.

In a further example, an insertion device includes a handle. The handle consists essentially of a rounded ball-head having a port extending from a first side of the rounded ball-head and a grip depending from the rounded ball-head, the grip including a pair of fluid ports and a plug extending from the grip in a direction opposite that of the rounded ball-head. The insertion device further includes a shaft having a proximal end disposed within the rounded ball-head and extending from a second side of the ball-head in a direction opposite the first side of the rounded ball-head. A cap is coupled to a distal end of the shaft and the cap includes a camera and a light source coupled thereto.

Examples of the insertion device include one or more of the following features. A proximal end of a working channel is coupled to the port and extends distally through a proximal end of the shaft and towards the cap. The handle includes a plug extending from a portion of the extension of the handle opposite the ball-head, and wherein a cable or a bundle of cables have a first end coupled to at least one of the camera and light source and a second end coupled to the plug.

Both the foregoing general description and the following detailed description are exemplary and explanatory only and are not restrictive of the features, as claimed. As used herein, the terms "comprises," "comprising," or other variations thereof, are intended to cover a non-exclusive inclusion such that a process, method, article, or apparatus that comprises a list of elements does not include only those elements, but may include other elements not expressly listed or inherent to such a process, method, article, or apparatus. Additionally, the term "exemplary" is used herein in the sense of "example," rather than "ideal." As used herein, the terms "about," "substantially," and "approximately," indicate a range of values within +/- 5% of a stated value.

### BRIEF DESCRIPTION OF THE DRAWINGS

The accompanying drawings illustrate exemplary features of the present disclosure and together with the description, serve to explain the principles of the disclosure.
FIG. 1 illustrates an exemplary disposable insertion device according to aspects of the disclosure;
FIG. 2 illustrates a partial isometric view of a cap of the exemplary insertion device of FIG. 1;
FIG. 3 illustrates a partial cross-sectional view of the insertion device of FIG. 1;
FIG. 4 illustrates a cross-sectional view of a shaft of the insertion device of FIG. 1, according to aspects of the disclosure; and
FIG. 5 illustrates a cross-sectional view of a shaft of an insertion device, according to additional aspects of the disclosure.

### DETAILED DESCRIPTION

Examples of the present invention relate to medical systems and devices for diagnosing and/or treating internal areas of a subject's body. The medical system includes an insertion device and one or more medical devices operably coupled thereto for introduction of an end-effector or other object through the insertion device.

Reference will now be made in detail to examples of the present invention described above and illustrated in the accompanying drawings. Wherever possible, the same reference numbers will be used throughout the drawings to refer to the same or like parts.

The terms "proximal" and "distal" are used herein to refer to the relative positions of the components of an exemplary medical device or insertion device. When used herein, "proximal" refers to a position relatively closer to the exterior of the body or closer to an operator using the medical device or insertion device. In contrast, "distal" refers to a position relatively further away from the operator using the medical device or insertion device, or closer to the interior of the body.

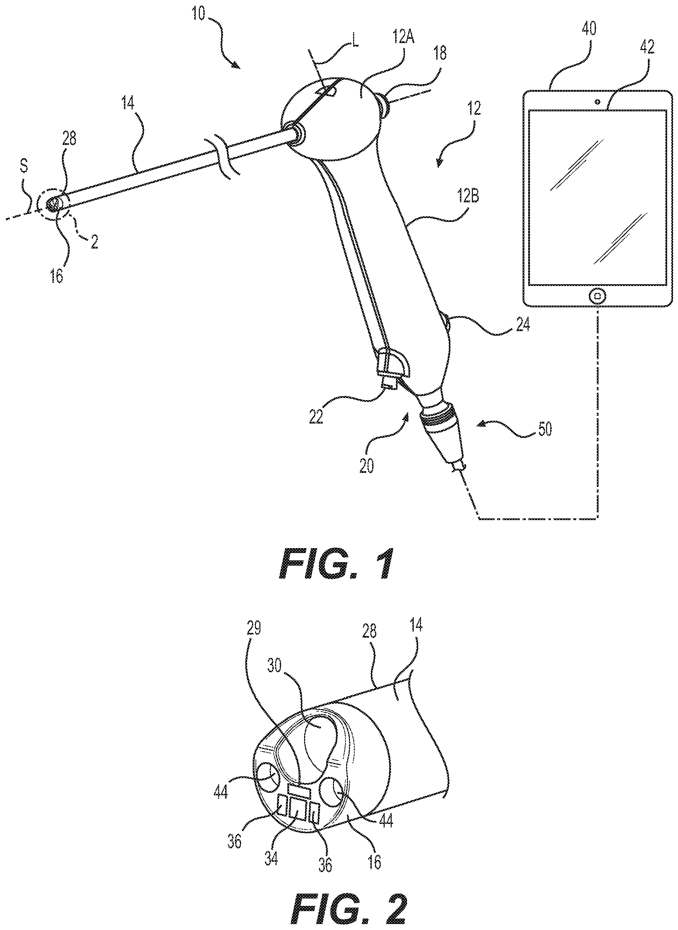

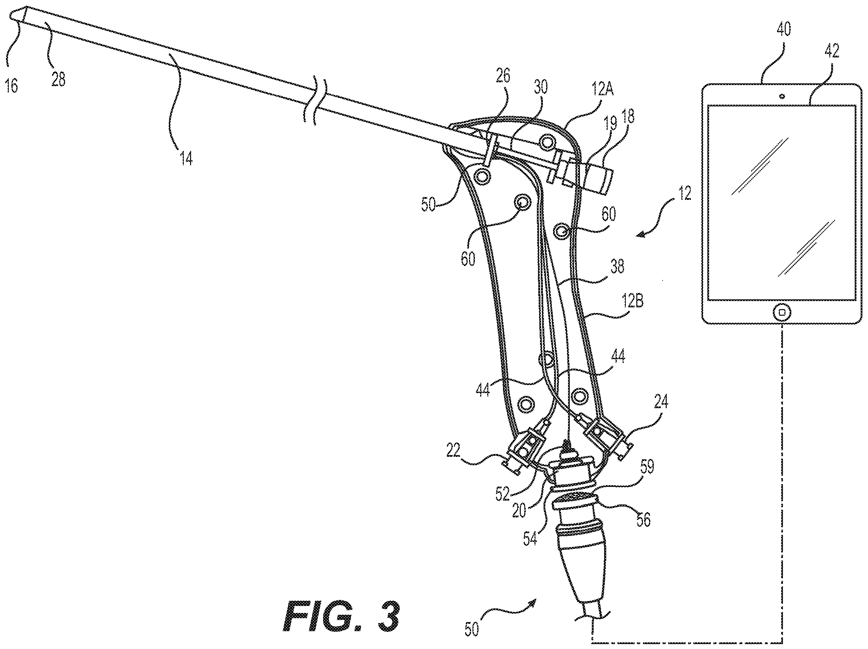

FIGS. 1 and 2 illustrate an insertion device 10 including a hollow handle 12 coupled to a shaft 14 terminating in a cap 16. Handle 12, as will be described in further detail below, is a hollow or empty handle in which only shaft 14, first and second tubes 44, a working channel 30, a cable/bundle 38, ports 18, 22, and 24, and a plug 20 extends or resides therein. Additionally, one or more molding remnants, flashings, or handle connection members 60 (FIG. 3) for connecting portions of the handle together may be present within hollow handle 12. For example, connection members 60 may be used to hold the two handle halves together after assembly. They may contain screws, function as adhesive bonding pads, ultrasonic weld points, friction fit points, and/or snap fits. Alternatively, connection members 60 may be absent from insertion device 10. In such a manner, hollow handle 12 is devoid of any additional functional components (e.g., heat sinks, circuit boards, illumination sources, steering cables or actuators, processing equipment, and/or additional circuitry) found in prior art devices. As such, hollow handle 12 is relatively inexpensive, light, and uncluttered or unencumbered as compared to prior art handles. In other words, hollow handle 12 refers to a device containing only working channel 30, first and second tubes 44, cable/bundle 38, plug 20, and ports 18, 22, and 24, and does not include heat sinks, circuit boards, illumination sources, steering cables or actuators, processing equipment or additional circuitry, as will be described in further detail below. That is, handle 12 may consist essentially of shaft 14, first and second tubes 44, working channel 30, a cable/bundle 38, ports 18, 22, and 24, and a plug 20 while not including any additional hardware components such as, e.g., heat sinks, circuit boards, illumination sources, steering cables or actuators, processing equipment, and/or additional circuitry. Additionally, while hollow handle 12 is generally devoid of a light source housed therein, in an alternative embodiment, a partially hollow handle 12 may include one or more LEDs 36 therein, and include one or more optical fibers extending along shaft 14 for conveying light distally of cap 16.

Cap 16 includes a rounded circumferential edge and is angled so as to be atraumatic to tissue. Additionally, cap 16 may be coupled (e.g., removably or permanently) to shaft 14 in any appropriate manner such as, for example, snap fit, interference fit, adhesives, welding, molding, etc. Insertion device 10 includes any device configured to allow an operator to perform medical diagnoses and/or treatments on a subject. For example, insertion device 10 includes any device configured to allow a user to access and view internal areas of a subject's body. For example, insertion device 10 may be inserted into any portion of a urinary tract, such as a ureter, a gastrointestinal lumen, such as an esophagus; a vascular lumen; and/or an airway.

According to aspects of the present disclosure, insertion device 10 is a ureteroscope. In some contemplated examples, insertion device 10 is a sterile, single-use, and completely disposable ureteroscope. That is, upon completion of a medical procedure, insertion device 10, including hollow handle 12, shaft 14, and cap 16 may be disconnected from any appropriate console or power source (e.g., computer 40) and thrown away. As such, concerns of cross-contamination or insufficient sterilization of insertion device 10 may be alleviated. Other types of devices, however, may be substituted for the ureteroscope, including, as examples, a hysteroscope, a uteroscope, a bronchoscope, a cystoscope, and similar devices. Any such devices may be single-use and completely disposable.

Hollow handle 12 of insertion device has any shape suitable for gripping and controlling insertion device 10. For example, hollow handle 12 may have an ergonomic shape designed to be held comfortably in the hand, e.g., the palm of the hand of a medical professional. For example, as shown in FIG. 1, hollow handle 12 includes an enlarged spherical, or substantially spherical ball-head 12A coupled to shaft 14 and a pistol grip or extension 12B depending therefrom. As shown, ball-head 12A includes a single (i.e., only one) port 18 on a proximal side thereof (e.g., a side of hollow handle 12 closest to a body of the medical professional when being held by the medical professional, and/or a side of ball-head 12A opposite the side from which shaft 14 extends), as will be described in further detail below. Additionally, a selectively coupleable and uncouplable plug 20 and ports 22 and 24 positioned on extension 12B are located on a proximal end thereof, e.g., on an end of extension 12B opposite the position of the ball-head 12A, as will be described in further detail below. In such a manner, a medical professional grips hollow handle 12 along either the enlarged ball-head 12A or the extension 12B, depending on his/her preference. For example, in some arrangements, the medical professional may grip or hold hollow handle 12 by placing or wrapping the palm of his or her hand on or around a top surface (e.g., a surface of ball-head 12A opposite the extension 12B) of ball-head 12A, thereby avoiding interference with port 18 of ball-head 12A. Additionally or alternatively, in some arrangements, the medical professional grips or holds hollow handle 12 by placing or wrapping the palm of his or her hand on or around the extension between the ball-head 12A and plug 20 and ports 22 and 24, such that interference with plug 20 and ports 18, 22, and 24, may be avoided.

As shown in FIG. 1, hollow handle 12 is bilaterally symmetric about a plane extending along the longitudinal axis L of hollow handle 12 and includes a clam shell arrangement. Shaft 14 extends along a longitudinal shaft axis S from a proximal end 26 within hollow handle 12 (FIG. 3) to a distal end 28 coupled to cap 16. As shown in FIG. 3, proximal end 26 of shaft 14 is fixedly secured via attachment mechanism 50 (e.g., screws, bolts, or the like) to hollow handle 12 such that shaft is fixedly coupled (e.g., rotationally and axially immovably attached) to hollow handle 12. Shaft 14 is a rigid (e.g., non-bendable, deflectable, etc.) shaft. For example, shaft 14 may be comprises of one or more of the following materials: stainless steel, titanium, rigid plastic, glass fiber reinforced polymer resin (fiberglass), and hard coat anodized aluminum. Additionally, shaft 14 has a length extending from proximal end 26 to distal end 28 of between about 230 mm to about 335 mm.

As noted above, hollow handle 12 includes a plurality of ports. For example, port 18 extends from ball-head 12A parallel or aligned with longitudinal axis S and communicates with a working channel 30 extending through shaft 14 and tip 16, as shown in FIGS. 2 and 3. That is, working channel 30 extends from port 18 through shaft 14 and terminates in cap 16. Working channel 30 extends along an axis offset and parallel with longitudinal axis S and includes a radial size (e.g., diameter) smaller than a radial size (e.g., diameter) of shaft 14. Additionally, working channel 30 includes a radial size (e.g., diameter) larger than a radial size (e.g., diameter) of any other channel (e.g., first and second tubes 44, as will be described in further detail below) of insertion device 10. Additionally, although working channel 30 is illustrated and described as having a circular cross-sectional shape, the disclosure is not so limited. Rather, working channel 30 may have an oval, semicircular, or any other cross-sectional shape that defines an inner passage way therethrough. Port 18 may facilitate the insertion of one or more medical device(s) through working channel 30 to distal end 28 of shaft 14 and cap 16. For example, port 18 may comprise a medical (e.g., threaded) or slip luer port through which an end-effector, control member, and/or sheath of a medical device (not shown) may be delivered into and/or through working channel 30. Port 18 may further include a one-way valve 19 (FIG. 3) which allows the passage (insertion and removal) of a medical device therethrough but prevents or limits the outlet of fluid from working channel 30.

As shown in FIG. 2, insertion device 10 includes an imaging assembly including a digital camera 34 at a distal end of cap 16. For example, camera 34 may be at a distalmost tip of cap 16 and/or may be at least partially embedded within cap 16. Camera 34 may view an area distal to the distal end 28 of shaft 14 and cap 16. Camera 34 may have any appropriate resolution to capture images and/or full-motion video images in digital or any other suitable format. For example, camera 34 may include a pixel count greater than about 20,000 pixels and less than about 80,000 pixels. For example, camera 34 may have a pixel count of about 62,500. Camera 34 may include a field of view of at least 80°. Additionally, camera 34 may include all those components (e.g., sensors, filters, signal converters) necessary for receiving and transmitting an image signal to a computer 40 via a cable 38, as will be described in further detail below. That is, camera 34 may contain a lens and a digital sensor to transmit a signal via cable 38 to computer 40 for processing and display.

Additionally, insertion device 10 includes an illumination unit including one or more (e.g., two) light-emitting diodes ("LED") 36. LEDs 36 may be at a distalmost tip of cap 16 and/or may be at least partially embedded within cap 16. As shown, LEDs 36 may be located on opposite sides of camera 34 and may emit light upon receipt of an appropriate power supply. LED 36 may include, for example, a high-power sub 2mm LED with a CCT range of 5000-6500K. Any other suitable LED may be used. Further, insertion device 10 includes a pressure sensor 29 at least partially embedded within cap 16. Pressure sensor 29 may be arranged to directly measure body cavity pressure of a patient. Pressure sensor 29 may include a piezoresistive pressure die (such as, e.g., the P330 Series 1F Absolute Pressure Sensor Die from Nova Sensor).

A bundle of cables 38 (or alternatively, a single cable 38 adapted for conveyance of both power and data) may be coupled to one or more of camera 34, LEDs 36, and pressure sensor 29 and extend proximally along shaft 14 between an outer wall of working channel 30 and an inner wall of shaft 14, and connect to plug 20. Plug 20 includes an electrical connector to connect hollow handle 12, including cable/bundle 38, to an umbilicus 50 for facilitating electrical connections and functions, such as transferring data to/from camera 34, pressure sensor 29, and/or powering LEDs 36. As shown in FIG.3, for example, plug 20 may include one or more terminals 52 fixedly coupled to (e.g., soldered to) cable/bundle 38. Additionally, plug 20 may include an adaptor 54 for selectively coupling and uncoupling from an adaptor 56 of umbilicus 50.

To facilitate secure connection or mating between adaptors 54 and 56, adaptors 54 and 56 may be magnetically attracted toward one another, or threadbly connected to maintain contact therebetween, or use a method of interfering parts (snap or lever release connection) to hold the mating halves together. Additionally or alternatively, at least one of adaptors 54 and 56 may include one or more leads (not shown) arranged to mate or otherwise connect with one or more receptacles 59 on the other of adaptors 54 and 56 so as to convey signals (e.g., power and/or data) from/to cable/bundle 38 to or from umbilicus 50, which is in turn coupled to a computer 40.

Computer 40 may include a smartphone, tablet computer, laptop computer, desktop computer, and/or any other suitable computing device. Computer 40 may include a display 42, as well as other electronic components (not shown), such as a central processing unit, memory, video and graphics cards, wireless and wired networking devices, audio devices, one or more input/output ports, a power supply, and/or any other suitable computer features. Display 42 may include a touch screen for displaying image data, and for receiving inputs or commands from a user. User interaction may be directed toward aspects of image capture, video capture, brightness control, mode controls, narrow band imaging toggle, and/or any other controls that may be part of a typical ureteroscopy procedure. Computer 40 may include imaging electronics configured to process and/or transfer the image data, display the image data on display 42 for viewing by a user, and send signals to camera 34 and LEDs 36.

Each of ports 22 and 24 may include a luer (or other mating configuration) fluidly coupled with one of first and second channels, passages, or tubes 44 extending through shaft 14 and tip 16, as shown in FIGS. 2 and 3. That is, each tube 44 extends from one of ports 22 and 24, through shaft 14, and terminates in cap 16. Any one or both of tubes 44 may be fluidly coupled with a source of irrigation fluid, a source of aspiration fluid (e.g., negative pressure), a source of medicament for infusion, etc. Additionally, in some arrangements one or both of tubes 44 may receive pressure sensor 29, therein. For example, in one arrangement, a first tube 44 is fluidly coupled with a source of irrigation fluid (not shown) while a second tube 44 is fluidly coupled with a source of aspiration fluid (not shown). In another arrangement, at least the first tube 44 is fluidly coupled with a source of irrigation fluid (not shown) while a second tube 44 is fluidly coupled with a source of medicament. While in some exemplary arrangements, only two tubes 44 are employed, in further arrangements, more or less tubes 44 may be used. For example, in some arrangements, a single (e.g., only one) tube 44 may be employed, while in still further arrangements, three or more tubes 44 may be used.

In any such arrangement, ports 22 and 24 are located on a proximal end thereof of extension 12B opposite the position of the ball-head 12A. As shown in FIGS. 1 and 3, ports 22 and 24 may be circumferentially spaced 180° apart about the longitudinal axis L of hollow handle 12. In other arrangements, however, ports 22 and 24 may be spaced apart at any appropriate circumferential angle, such as, between about 20° and about 340° arranged about longitudinal axis L of hollow handle 12.

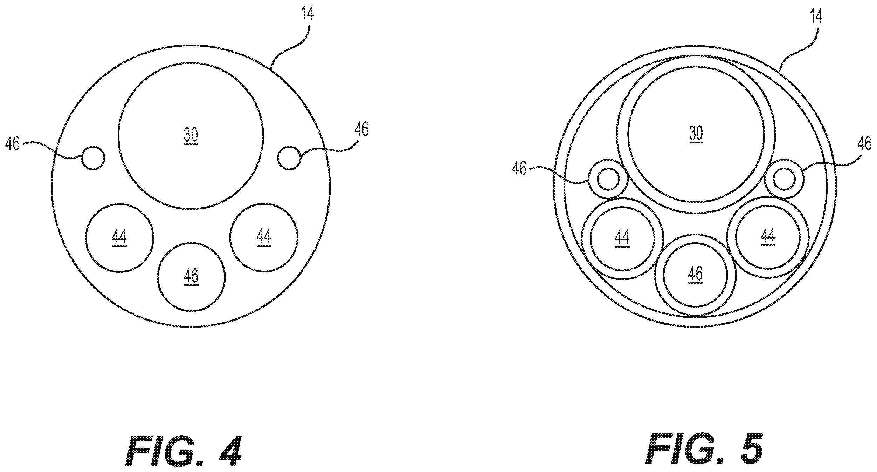

Shaft 14 of FIGS. 1-3 may be arranged as either a multi-lumen extrusion (FIG. 4) or as individual and discrete lumen or tubes secured together via, e.g., adhesives, welding, or the like (FIG. 5). In the arrangement shown in FIG. 5, spaced between adjacent lumens may optionally be filled with holding substance such as, for example, epoxy, adhesives, or the like. As shown in each of FIGS. 4 and 5, shaft 14 includes working channel 30 and first and second tubes 44. Optionally, shaft 14 may further include any or one or more cable lumens 46 (e.g., three cable lumens 46 are shown). In the case of the partial hollow handle in which LEDs 36 are located within handle 12, one or more of the cable lumens 46 may house or otherwise receive an optical fiber or the like for conveying light distally of shaft 14. Also, while each of lumens 30, 44, and 46 are illustrated as having a circular cross-section, the disclosure is not so limited. Rather, any one or more of lumens 30, 44, or 46 may have any appropriate shape including, for example, square, ovular, polygonal, and irregular shapes, or combinations thereof.

In use, a medical professional grasps hollow handle 12 (e.g., either via ball-head 12A or extension 12B) and direct shaft 14 to enter one or more body passages of a patient. If desired or necessary, the medical professional delivers one or more medical tools or devices (e.g., baskets, forceps, etc.) through port 18 and working channel 30 so as to perform a medical procedure. Additionally, if it is determined desirable or necessary, the medical professional causes one or more of an irrigation fluid, aspiration fluid, or medicament infusion to be delivered through tubes 44 and distally of shaft 14. Once a medical procedure has been completed, the medical professional retracts shaft 14 from the patient, uncouples umbilicus 50 from plug 20, and discards or throws away the entirety of hollow handle 12, including plug 20 and shaft 14. In such a manner, cross-contamination and insufficient sterilization concerns are alleviated.

## Claims

1. An insertion device (10), comprising:
a hollow handle (12);
a shaft (14) extending distally of the hollow handle (12); and
a cap (16) coupled to a distal end of the shaft (14), the cap (16) including a camera (34) and an LED (36) coupled thereto,
wherein the hollow handle (12) includes a rounded ball-head (12A) and a grip (12B) depending therefrom, wherein the shaft (14) extends from a location within the ball-head (12A) and through a side of the ball-head (12A) of the hollow handle (12), and wherein a port (18) extends from a side of the ball-head (12A) of the hollow handle (12) opposite the side from which the shaft (14) extends.

2. The insertion device (10) of claim 1, wherein the shaft (14) is a rigid shaft (14).

3. The insertion device (10) of any one of the preceding claims, wherein the shaft (14) includes a multi-lumen extrusion.

4. The insertion device (10) of any one of claims 1 or 2, wherein the shaft (14) includes a plurality of discrete lumens (30, 44, 46) fixedly coupled together.

5. The insertion device (10) of any one of the preceding claims, wherein the shaft (14) extends along a longitudinal shaft (14) axis S from a proximal end within hollow handle (12) to a distal end coupled to cap (16), wherein the port (18) extends from ball-head (12A) parallel or aligned with longitudinal axis S and communicates with a working channel (30) extending through the shaft (14) and terminating in the cap (16).

6. The insertion device (10) of any one of the preceding claims, wherein the hollow handle (12) is devoid of any circuitry.

7. The insertion device (10) of any one of the preceding claims, wherein the hollow handle (12) includes a plug (20) extending from a portion of the grip (12B) of the hollow handle (12) opposite the ball-head (12A).

8. The insertion device (10) of claim 7, wherein the plug (20) is selectively coupleable and uncouplable from an umbilicus connector (50).

9. The insertion device (10) of any one of claims 7 and 8, wherein the handle (12) includes two fluid ports (22, 24) proximate the plug (20).

10. The insertion device (10) of claim 9, wherein each of the two fluid ports (22, 24) is fluidly coupled to a tube (44) extending through the hollow handle (12), through the shaft (14), and toward the cap (16).

11. The insertion device (10) of claim 8, wherein the umbilicus (50) is coupled to a computer (40).

12. The insertion device (10) of any one of the preceding claims, wherein the LED (36) is a first LED (36), the insertion device (10) further including a second LED (36) disposed on the cap (16).

13. The insertion device (10) of claim 12, wherein each of the first and second LEDs (36) is disposed on opposing sides of the camera (34).

14. The insertion device (10) of claim 1, wherein a working channel (30) extends through and proximally of a proximal end of the shaft (14).

## Patentansprüche

1. Insertionsvorrichtung (10), umfassend:
einen hohlen Griff (12);
einen Schaft (14), der sich distal von dem hohlen Griff (12) erstreckt; und
eine Kappe (16), gekoppelt an ein distales Ende des Schafts (14), wobei die Kappe (16) eine Kamera (34) und eine LED (36), daran gekoppelt, umfasst,
wobei der hohle Griff (12) einen abgerundeten Kugelkopf (12A) und einen Griff (12B), daran anhängend, umfasst, wobei sich der Schaft (14) von einer Stelle innerhalb des Kugelkopfs (12A) und durch eine Seite des Kugelkopfs (12A) des hohlen Griffs (12) erstreckt, und wobei sich ein Anschluss (18) erstreckt von einer Seite des Kugelkopfs (12A) des hohlen Griffs (12), gegenüber der Seite, von der sich der Schaft (14) erstreckt.

2. Insertionsvorrichtung (10) nach Anspruch 1, wobei der Schaft (14) ein starrer Schaft (14) ist.

3. Insertionsvorrichtung (10) nach einem der vorangehenden Ansprüche, wobei der Schaft (14) eine Mehrlumenextrusion umfasst.

4. Insertionsvorrichtung (10) nach einem der Ansprüche 1 oder 2, wobei der Schaft (14) eine Vielzahl fest miteinander verbundener, diskreter Lumina (30, 44, 46) umfasst.

5. Insertionsvorrichtung (10) nach einem der vorangehenden Ansprüche, wobei sich der Schaft (14) entlang einer Längsachse S des Schafts (14) von einem proximalen Ende innerhalb des hohlen Griffs (12) zu einem distalen Ende, gekoppelt an die Kappe (16), erstreckt, wobei sich der Anschluss (18) vom Kugelkopf (12A) parallel zur Längsachse S oder ausgerichtet auf sie erstreckt und sich im Austausch mit einem Arbeitskanal (30), der sich durch den Schaft (14) erstreckt und in der Kappe (16) endet, befindet.

6. Insertionsvorrichtung (10) nach einem der vorangehenden Ansprüche, wobei der hohle Griff (12) frei von jeglicher Schaltung ist.

7. Insertionsvorrichtung (10) nach einem der vorangehenden Ansprüche, wobei der hohle Griff (12) einen Stopfen (20) umfasst, der sich von einem Abschnitt des Handgriffs (12B) des hohlen Griffs (12) gegenüber dem Kugelkopf (12A) erstreckt.

8. Insertionsvorrichtung (10) nach Anspruch 7, wobei der Stopfen (20) wahlweise mit einem Umbilikus-Verbinder (50) verbindbar und von ihm trennbar ist.

9. Insertionsvorrichtung (10) nach einem der Ansprüche 7 und 8, wobei der Griff (12) zwei Fluidanschlüsse (22, 24) in der Nähe des Stopfens (20) umfasst.

10. Insertionsvorrichtung (10) nach Anspruch 9, wobei jeder der zwei Fluidanschlüsse (22, 24) fluidisch an eine Röhre (44) gekoppelt ist, die sich durch den hohlen Griff (12), durch den Schaft (14) und in Richtung der Kappe (16) erstreckt.

11. Insertionsvorrichtung (10) nach Anspruch 8, wobei der Umbilikus (50) an einen Computer (40) gekoppelt ist.

12. Insertionsvorrichtung (10) nach einem der vorangehenden Ansprüche, wobei die LED (36) eine erste LED (36) ist und die Insertionsvorrichtung (10) ferner eine an der Kappe (16) angeordnete zweite LED (36) umfasst.

13. Insertionsvorrichtung (10) nach Anspruch 12, wobei jede von der ersten und zweiten LED (36) auf gegenüberliegenden Seiten der Kamera (34) angeordnet ist.

14. Insertionsvorrichtung (10) nach Anspruch 1, wobei sich ein Arbeitskanal (30) durch ein proximales Ende des Schafts (14) und proximal zu ihm erstreckt.

## Revendications

1. Dispositif d'insertion (10), comprenant :
un manche creux (12) ;
un arbre (14) qui s'étend de façon distale par rapport au manche creux (12) ; et
un capuchon (16) qui est couplé à une extrémité distale de l'arbre (14), le capuchon (16) incluant une caméra (34) et une diode électroluminescente/LED (36) qui lui est couplée ;
dans lequel le manche creux (12) inclut une tête en forme de rotule arrondie (12A) et un moyen de préhension (12B) qui en dépend, dans lequel l'arbre (14) s'étend depuis une localisation à l'intérieur de la tête en forme de rotule (12A) et au travers d'un côté de la tête en forme de rotule (12A) du manche creux (12), et dans lequel un orifice (18) s'étend depuis un côté de la tête en forme de rotule (12A) du manche creux (12) à l'opposé du côté depuis lequel l'arbre (14) s'étend.

2. Dispositif d'insertion (10) selon la revendication 1, dans lequel l'arbre (14) est un arbre rigide (14).

3. Dispositif d'insertion (10) selon l'une quelconque des revendications précédentes, dans lequel l'arbre (14) inclut une extrusion à multiples lumières.

4. Dispositif d'insertion (10) selon l'une quelconque des revendications 1 ou 2, dans lequel l'arbre (14) inclut une pluralité de lumières discrètes (30, 44, 46) qui sont couplées ensemble de façon fixe.

5. Dispositif d'insertion (10) selon l'une quelconque des revendications précédentes, dans lequel l'arbre (14) s'étend suivant un axe longitudinal S de l'arbre (14) depuis une extrémité proximale à l'intérieur du manche creux (12) jusqu'à une extrémité distale qui est couplée au capuchon (16), dans lequel l'orifice (18) s'étendu depuis la tête en forme de rotule (12A) parallèlement à l'axe longitudinal S ou en alignement avec ce même axe longitudinal et il communique avec un canal de travail (30) qui s'étend au travers de l'arbre (14) et qui se termine à l'intérieur du capuchon (16).

6. Dispositif d'insertion (10) selon l'une quelconque des revendications précédentes, dans lequel le manche creux (12) est dénué de tout circuit.

7. Dispositif d'insertion (10) selon l'une quelconque des revendications précédentes, dans lequel le manche creux (12) inclut un bouchon (20) qui s'étend depuis une partie du moyen de préhension (12B) du manche creux (12) qui est opposée à la tête en forme de rotule (12A).

8. Dispositif d'insertion (10) selon la revendication 7, dans lequel le bouchon (20) peut, de façon sélective, être couplé à un connecteur ombilical (50) et peut, de façon sélective, en être découplé.

9. Dispositif d'insertion (10) selon l'une quelconque des revendications 7 et 8, dans lequel le manche creux (12) inclut deux orifices de fluide (22, 24) qui sont à proximité du bouchon (20).

10. Dispositif d'insertion (10) selon la revendication 9, dans lequel chacun des deux orifices de fluide (22, 24) est couplé en termes de fluide à un tube (44) qui s'étend au travers du manche creux (12), au travers de l'arbre (14) et en direction du capuchon (16).

11. Dispositif d'insertion (10) selon la revendication 8, dans lequel le connecteur ombilical (50) est couplé à un ordinateur (40).

12. Dispositif d'insertion (10) selon l'une quelconque des revendications précédentes, dans lequel la LED (36) est une première LED (36), le dispositif d'insertion (10) incluant en outre une seconde LED (36) qui est disposée sur le capuchon (16).

13. Dispositif d'insertion (10) selon la revendication 12, dans lequel chacune des première et seconde LED (36) est disposée sur des côtés opposés de la caméra (34).

14. Dispositif d'insertion (10) selon la revendication 1, dans lequel un canal de travail (30) s'étend au travers d'une extrémité proximale de l'arbre (14) et de façon proximale par rapport à cette même extrémité proximale.
